# EUROPÄISCHE PATENTSCHRIFT

(11) **EP 2 858 655 B1**
(45) Veröffentlichungstag und Bekanntmachung des Hinweises auf die Patenterteilung: **01.06.2016**
(21) Anmeldenummer: 13730152.9
(22) Anmeldetag: 10.06.2013
(51) Int. Cl.: A61K 47/26, A61P 29/00, A61P 1/00, A61K 36/53

(54) **Thymian Extrakte und deren Verwendung**
THYME EXTRACTS AND THEIR USE
EXTRAITS DE SERPOLET ET LEUR UTILISATION

(30) Priorität: 08.06.2012 EP 12171273
(43) Veröffentlichungstag der Anmeldung: 15.04.2015
(73) Patentinhaber: Finzelberg GmbH & Co. KG, 56626 Andernach (DE)
(72) Erfinder: WALBROEL, Bernd, 53639 Königswinter (DE); PISCHEL, Ivo, 53547 Rossbach (DE); FEISTEL, Björn, 56626 Andernach (DE)
(74) Vertreter: dompatent von Kreisler Selting Werner Partnerschaft von Patent- und Rechtsanwälten mbB
(86) Internationale Anmeldenummer: PCT/EP2013/061917
(87) Internationale Veröffentlichungsnummer: WO 2013/182709

(56) Entgegenhaltungen:
- EP-A1- 1 080 727
- WO-A1-2010/023422
- DE-A1- 4 213 167
- UA-A- 64 193
- PAVEL M ET AL: "Phytochemical and pharmacological research on some extracts obtained from Serpylli herba", FARMACIA 2011 ROMANIAN SOCIETY FOR PHARMACEUTICAL SCIENCES ROU, Bd. 59, Nr. 1, 2011, Seiten 77-84, XP009162420, ISSN: 0014-8237
- BASANT BALLABH* & O P CHAURASIA: "Medicinal plants of cold desert Ladakh used in the treatment of stomach disorders", INDIAN JOURNAL OF TRADITIONAL KNOWLEDGE, RESOURCES, NEW DELHI, NEW DELHI - INDIA, Bd. 8, Nr. 2, 1. April 2009 (2009-04-01), Seiten 185-190, XP018027507, ISSN: 0972-5938
- DATABASE WPI Week 200981 Thomson Scientific, London, GB; AN 2009-R61151 XP002682747, & JP 2009 276245 A (SHISEIDO CO LTD) 26. November 2009 (2009-11-26)
- RAAL AIN ET AL: "Content and composition of the essential oil of Thymus serpyllum L. growing wild in Estonia.", MEDICINA (KAUNAS, LITHUANIA) 2004 LNKD- PUBMED:15300002, Bd. 40, Nr. 8, 2004, Seiten 795-800, XP009162425, ISSN: 1648-9144

## Beschreibung

Die vorliegende Erfindung betrifft Extrakte aus Quendelkraut (Thymus serpyllum), ihre Herstellung und Verwendung.

Eine Verwendung von Thymian (Thymus spp.) ist besonders für kulinarische Zwecke, medizinisch aber auch bei Atemwegserkrankungen beschrieben.

Thymian ist eine kulinarische Pflanze und Heilkraut aus der Gattung Thymus. Thymian ist bekannt für seinen starken Geschmack und wird wegen seines Gehalts an Thymol angebaut, am besten an heißen, sonnigen Standorten mit gut durchlässigem Boden. Im Frühjahr gepflanzt, wächst er als Staude und kann durch Samen, Stecklinge oder durch Wurzelteilung vermehrt werden. Oft wird er auch wild gesammelt.

Thymian ist weltweit verbreitet und wird kulinarisch in den Küchen Europas, des nahen Ostens, Asiens, Indiens, Afrikas und Amerikas verwendet. Besonders wird Thymian zum Würzen von Fleisch, Suppen und Eintöpfen benutzt; dies als Einzelgewürz oder in Kräuter- und Gewürzmischungen. In den levantinischen Ländern als Gewürz Za'atar (arabisch für Thymian) ist Thymian als wichtige Zutat enthalten, sowie als Bestandteil des Bouquet garni und der herbes de Provence.

Thymian wird sowohl frisch als auch getrocknet gehandelt. Die frische Ware ist aus dem Treibhaus fast ganzjährig verfügbar und sehr aromatisch, geerntet aber nicht länger als eine Woche haltbar. Frischer Thymian wird in Bündeln der Zweige verkauft und besteht aus einem holzigen Stamm, der mit Blatt- oder Blütenständen besetzt ist. Je nach Rezept werden ganze Zweige oder die Blätter vom Stängel befreit verwendet. Getrockneter Thymian bewahrt sein Aroma besser als viele andere Kräuter.

Neben der kulinarischen Verwendung kommen dem, das Aroma gebenden ätherischen Öl verschiedene medizinische Funktionen zu. So enthält das Thymianöl des gemeinen Thymian (Thymus vulgaris) 20-54% Thymol, das als Antiseptikum beispielsweise den Hauptwirkstoff in verschiedenen Mundspülungen wie Listerine stellt. Früher wurde das Öl von Thymian auch in der Wundheilung in Verbänden oder topisch gegen verschiedene Pilze eingesetzt sowie als antibiotisch wirksames Desinfektionsmittel. Das ätherische Öl des Quendelkrauts (Thymus serpyllum) hingegen weist einen hohen Gehalt an Carvacrol, einem dem Thymol stereoisomeren Terpenphenol auf, mit analogem Wirkungsspektrum.

Wässrige Teeinfusionen des Thymiankrauts werden bei Husten und Bronchitis eingesetzt. Weitere medizinische Anwendungen sind Infektionen der Atemwege, wobei Thymian neben Kräutertee in Form von Tinkturen, Salbe, Sirupen oder durch Dampfinhalationen angewendet wird. Da Thymian antiseptisch wirkt, kann der Dekokt oder der Infus in Wasser nach dem Abkühlen sehr gut gegen Halsentzündungen eingesetzt werden, wenn mehrfach täglich damit gegurgelt wird. Dabei bewirkt das aufgenommene Thymol und andere flüchtige Bestandteile des Thymiankrauts, weil über die Lunge ausgeschieden (abgeatmet), aufgrund seiner Lipophilie eine Reduzierung der Viskosität des Bronchialschleimes, wodurch dieser leichter ausgeworfen werden kann. Darüber hinaus üben sie ihre antimikrobielle Wirkung im respiratorischen Trakt aus. Auch andere Infektionen und Wunden können mit Thymiandekokten behandelt werden.

Nahezu alle voranstehenden Eigenschaften und Verwendungen lassen sich für die regional verwendeten Thymiansorten beschreiben. Zu diesen drogenliefernde Arten zählen hauptsächlich Thymus capitatus, Thymus citriodorus, Thymus mastichina, Thymus pulegioides, Thymus serpyllum, Thymus vulgaris und Thymus zygis. Traditionell wird T. zygis mit T. vulgaris als gleichwertig betrachtet, spielt ökonomisch jedoch nur eine geringe Bedeutung. Aus dieser Gruppe sind vor allem T. vulgaris und T. serpyllum aus Anbau oder Wildsammlung in ökonomisch bedeutsamen Mengen zugänglich. In der Europäischen Pharmakopoe wird Quendel (Thymus serpylli L.)(Monographie 1891) als unerwünschte Verfälschung für den medizinal verwendeten Thymian (Thymus vulgaris L. + Thymus zygis) (Monographie 865) aufgeführt und vice versa. Dies ist der lange bekannten pharmakologischen Unterschiedlichkeit der Pflanzenarten geschuldet. Der Mindestgehalt an ätherischem Öl für die monographierten Drogen beträgt für Quendel mindestens 3 ml/kg, für Thymian mindestens 12 ml/kg.

Das pharmakologisch wertvolle ätherische Öl, welches unter der Monographie 1374 in der Ph.Eur. geführt wird, wird in dieser Konsequenz ausschließlich aus T. vulgaris oder T. zygis gewonnen.

Demzufolge wird in der Fachliteratur zumeist T. serpyllum und T. vulgaris getrennt behandelt und die Wirkung größtenteils dem ätherischen Ölanteil zugeschrieben. Hier werden neben dem bereits genannten kulinarischen Gebrauch auch kosmetische Parfüme-Anwendungen und insbesondere die Behandlung von Atemwegs- und Hustenerkrankungen genannt, aber auch die volkstümlichen, klinisch nicht belegten Anwendungen, wie äußerlich bei Hautleiden und -veränderungen, wie Wunden, Abszessen und Verbrennungen sowie innerlich als Antiseptikum bei entzündlichen Erkrankungen der Harnwege und des Darms u.a.m (HagerROM 2010).

Neben diesen klinisch nicht substantiierten Anwendungen von Thymianpräparaten in Hinsicht auf Magen-Darm-Leiden existiert eine Europäische Patentanmeldung EP1080727 (A1) - "Use of the extract of Thymus vulgaris for the preparation of a medicament for the treatment of Ulcerative Colitis and Crohn's disease" von ANASTASIOS EMMANOUILIDIS, welche Thymus vulgaris und dessen Inhaltsstoffe, insbesondere der ätherischen Öle, in Kombination mit Standardmedikamenten für diese Erkrankungen beansprucht und auf klinischen Fallstudien beruht.

Auch die Patentanmeldung DE4213167 (A1) - "Arzneimittel gegen Magenschleimhautentzündungen (Gastritis), Magen- und Duodenalulzera sowie Dyspepsie" von SUEKRETTIN GUELDUETUNA beschreibt die erfolgreiche Verwendung von Thymianöl. Hierin wird als Wirkweise auf die ätherisch-Öl-Komponenten Thymol / Carvacrol / Cymol und Cineol verwiesen und deren Aktivität gegen Helicobacter pylori.

Bereits in der Patentschrift EP0577481 (B1) - "Neue therapeutische Anwendungsform eines Thymianextraktes und in-vitro-Verfahren zur Inhibierung des Wachstums und der Ureaseaktivität von Helicobacter pylori" von ITZHAK NEEMAN findet man eine antibakterielle Aktivität wässriger Extrakte aus Thymus vulgaris, Thymus citriodorus und Coridothymus capitatus beschrieben.

Die Patentschrift UA 64193 A beschreibt Rezepturen von mehreren Medizinalpflanzen einschließlich wildem Thymian zur Behandlung und Prävention von Magen- und Duodenalgeschwüren sowie von chronisch kataranen Erkrankungen des Magen-Darm-Traktes.

Pavel M. et al. - "Phytochemical and pharmacological research on some extracts obtained from Serpylli herba", Farmacia 2011 Romanian Society for Pharmaceutical Sciences Rou, Bd. 59, Nr. 1, 2011, S. 77-84 - beschreibt die anti-inflammatorische Wirkung eines 1:2 hydroalkoholischen Flüssigextrakts einer anderen Thymianart, nämlich von Thymus Pulegioides L.

Basant Ballabh et al. - "Medicinal plants of cold desert ladakh used in the treatment of stomach disorders", Indian Journal of Traditional Knowledge, Resources, New Delhi, New Delhi - India, Bd. 8, Nr. 2, 2009, S. 185-190 - beschreibt die Verwendung einer Gesamtpflanzeninfusion von *Thymus serpyllum L.* bei Magenschmerzen und gastrointestinalen Problemen.

Die Patentschrift JP 2009276245 beschreibt ein Screening-Verfahren, nach dem Extrakte aus *Thymus serpyllum* erhalten werden; diese werden aber zur Verbesserung von Hautentzündungen, nicht von entzündlichen Darmerkrankungen verwendet.

Weiterhin befasst sich Raal, Ain et al. - "Content and composition of the essential oil of Thymus serpyllum L. growing wild in Estonia", Medicina (Kaunas, Lithuania) 2004, Bd. 40, Nr. 8, 2004, S. 795-800 - mit der Analyse des Gehalts und der Zusammensetzung von ätherischem Öl in wildem Thymian. Es werden jedoch keine Verwendungen offenbart.

Aufgabe der vorliegenden Erfindung war es, wirksame Zubereitungen von Thymian bereitzustellen und weitere Verwendungsmöglichkeiten aufzuzeigen.

Gelöst wird die Aufgabe durch neue Extrakte und neue Verwendungen von diesen Extrakten.

Im Rahmen systematischer Forschungstätigkeit wurden überraschenderweise neue Anwendungen von ausgewählten Thymiansorten gefunden. Somit können erfindungsgemäß, insbesondere wie nach dem hier vorliegenden Verfahren beschrieben, wirksame Thymianpräparationen gewonnen werden, die bei Entzündungen, beispielsweise chronisch-entzündlichen Verdauungstrakt- und Reizdarmleiden Einsatz finden.

Gegenstand der Erfindung ist daher die Verwendung von Extrakten aus Thymus serpyllum L. enthaltend zusätzlich ein Präbiotikum, ausgewählt aus der Gruppe der wasserlöslichen Kohlenhydrate zur Behandlung bei entzündlichen Erkrankungen des Intestinalbereichs, ausgewählt aus Reizdarmsyndrom (IBS) entzündlicher Darmerkrankung (IBD), lymphozytäre Colitis, Colitis ulcerosa, Divertikulitis, Duodenitis und Morbus Crohn.

Der Verdauungstrakt umfasst die Mundhöhle, den Pharynx, die Speiseröhre und den Magen-Darm-Trakt.

In einer Ausführungsform ist die entzündliche Erkrankung ein Reizdarmsyndrom (IBS) oder eine entzündliche Darmerkrankung (IBD).

In einer anderen Ausführungsform sind die entzündlichen Erkrankungen ausgewählt aus der Gruppe Lymphozytäre Colitis, Colitis ulcerosa, Divertikulitis, Duodenitis und Morbus Crohn.

Gegenstand der Erfindung ist weiterhin ein Extraktionsverfahren, mit dem besonders wirksame Extrakte erhalten werden können mit folgenden Schritten:
a. Bereitstellen einer Droge aus oberirdischen Pflanzenteilen von Thymus serpyllum L.
b. Optionalem Trocknen der Droge
c. Optionales Zerkleinern der Droge
d. Optionalem Rebeln der Blätter und Reduzierung des Stengelanteiles auf kleiner 2 Gew-%
e. Optionales Bedämpfen der Droge und Reduzierung des ätherischen Öles auf weniger als 0,5 ml/kg bezogen auf die getrocknete Droge
f. Extraktion der Droge mit Extraktionsmittel
g. Zumindest teilweises Entfernen des Extraktionsmittels unter Gewinnung eines Dickextraktes
h. Optionalem Entfetten mit flüssigen Lösemitteln
i. Optionale thermische Entkeimung
j. Hinzufügen eines Trocknungshilfsstoffes, wobei der Trocknungshilfsstoff ausgewählt wird aus der Gruppe der präbiotischen wasserlöslichen Kohlenhydrate, besonders bevorzugt aus der Gruppe der Fructo-Oligosaccharide
k. Trocknung unter Erhalt eines Extraktes.

Bevorzugt wird dabei mindestens einer der Schritte e, h oder i durchgeführt.

Überaschenderweise wurde gefunden, dass die erfindungsgemäß hergestellten Extrakte wirksam gegen entzündliche Erkrankungen eingesetzt werden können, selbst wenn die Komponenten des ätherischen Öles während der Herstellung des Extraktes weitestgehend entfernt werden. Dies kann bevorzugt entweder durch eine Bedämpfung der Droge vor der Extraktion erfolgen, oder durch eine Entfettung mittels organischen Lösemittels, oder im Rahmen der thermischen Entkeimung.

Geeignete Extraktionsmittel sind insbesondere ausgewählt aus der Gruppe Wasser, Alkohol, Keton, Ester, Ether oder überkritischer Gase oder deren Mischungen.

Bevorzugt wird als Extraktionsmittel Wasser oder eine Mischung von Wasser und Alkohol eingesetzt. Bevorzugt liegen im Falle von wässrigen Extraktionsmitteln die Alkoholgehalte im Bereich von 0 Gew.-% bis 70 Gew.-%, mehr bevorzugt 0 Gew.-% bis 40 Gew.-% oder 5 Gew.-% bis 40 Gew.-%. Als Alkohole sind insbesondere Ethanol, aber auch Methanol, n-Propanol oder Isopropanol besonders geeignet. Auch die Verwendung von CO₂ als Extraktionsmittel ist bevorzugt.

Extraktionen bei einer Temperatur von 20°C bis 100°C, bevorzugt bei 50°-90°C, besonders bevorzugt bei 60 - 80°C sind besonders geeignet, sind aber abhängig vom gewählten Extraktionsmittel.

Die eingesetzte Droge entspricht bevorzugt der Monographie "Serpylli herba" der europäischen Pharmakopoe.

Eine Trocknung durch Sprühtrocknung oder Unterdrucktrocknung ist bevorzugt.

Gegenstand der Erfindung ist auch ein Extrakt von oberirdischen Pflanzenteilen von Thymus serpyllum L sowie insbesondere der durch das oben beschriebene Verfahren erhältliche Extrakt oder eine entsprechende Extraktzubereitung.

Gegenstand der Erfindung ist auch eine Pharmazeutische Zubereitung, ein Medizinprodukt oder Lebensmittel enthaltend einen Extrakt von Thymus serpyllum L.

Bevorzugt enthält dieser Extrakt oder diese Extraktzubereitung einen Gehalt an ätherischem Öl von höchstens 0,5 Gew-%, bezogen auf den getrockneten Extrakt, bevorzugt höchstens 0,1 Gew-%, besonders bevorzugt höchstens 0,01 Gew-%. Der Gehalt an ätherischen Ölen in der Extraktzubereitung wird gemäß der Methode des Europäischen Arzneibuches (Ph.Eur. 2.8.12) analog der Methodik zur Bestimmung von ätherischem Öl in Pflanzendrogen bestimmt.

Entsprechend der guten Verträglichkeit der bisher bekannten Thymianpräparationen, war es Ziel der vorliegenden Erfindung, einen neuartigen Extrakt bereitzustellen, der sich für eine Verwendung im gesamten Bereich entzündlicher Erkrankungen eignet. Lokale anti-inflammatorische Effekte können gegebenenfalls entlang des gesamten Verdauungstraktes hilfreich bei der Behandlung oder Prophylaxe entzündlicher Erkrankungen sein. Gingivitis, Parodontitis, Pharyngitis, Ösophagitis und Gastritis beschreiben die entzündlichen Erkrankungen des oberen Teils des Verdauungstraktes. Lymphozytäre Colitis, Colitis ulcerosa, Divertikulitis, Duodenitis und Morbus Crohn sind - neben IBD und IBS - die bekanntesten Vertreter entzündlicher Erkrankungen des Intestinalbereichs. Erkrankungen des Intestinalbereichs sind die zum Darmkanal gehörenden Erkrankungen.

Für analytische Untersuchungen aus Thymianpräparaten werden auf Grund der hohen Flüchtigkeit der ätherisch-ÖI-Komponenten auch andere Stoffgruppen herangezogen. Hierunter ist als prominentester Vertreter der in Lamiaceen ubiquitär vorkommende Gerbstoff Rosmarinsäure zu nennen. Diese sollte bevorzugt für einen möglichst einfachen analytischen Vergleich der Extraktqualitäten mindestens einen Gehalt von 0,5 Gew.-% Rosmarinsäure enthalten. Mehr ist natürlich einfacher nachweisbar, weshalb eine minimale Grenzkonzentration von 1,5 Gew.-% Rosmarinsäure - bezogen auf den getrockneten nativen Extrakt - realisierbar erscheint.

Daher sind Extrakte, die mindestens 0,5 Gew.-% Rosmarinsäure, bevorzugt mindestens 1 Gew.-% Rosmarinsäure und noch mehr bevorzugt mindestens 1,5 Gew.-% Rosmarinsäure enthalten, besonders bevorzugt.

In einer bevorzugten Ausführungsform der Erfindung liegt der Gehalt an Thymol plus Carvacrol bei < 0,01 Gew.-% bezogen auf den getrockneten nativen Extrakt. Alle Angaben beziehen sich auf den nativen getrockneten Extrakt, d.h. den Extrakt ohne Zusatz von Hilfsstoffen.

Der erfindungsgemäße Extrakt wird zusammen mit einem Präbiotikum bereitgestellt und verwendet, wobei das Präbiotikum aus der Gruppe der wasserlöslichen Kohlenhydrate ausgewählt wird.

Unter den weiteren wirksamen Komponenten bei chronisch-entzündlichen Verdauungstrakt- und Reizdarmleiden werden oft präbiotische Stoffe genannt.

Präbiotika sind human-unverdauliche Nahrungsbestandteile, die durch das Wachstum und die Aktivitätssteigerung von Bakterien im Verdauungssystem die Gesundheit fördern.

Sie sind funktionelle Lebensmittel und als solche im Jahr 1995 von Marcel Roberfroid beschrieben worden. Typischerweise sind Präbiotika Kohlenhydrate (wie Oligosaccharide) und als wasserlösliche Ballaststoffe eingestuft, die meist der Gruppe der Fructooligosaccharide (z.B. Oligofructose und Inuline) angehören.

Man unterscheidet Präbiotika nach ihrer Kettenlänge zwischen kurzkettig mit 2-8 Zuckerbausteinen und langkettig, wie beispielsweise Inulin mit 9-64 Saccharid- Einheiten. In Abhängigkeit der Molekülgröße werden sie von unterschiedlichen Bakterienarten in verschiedenen Darmabschnitten als Substrate schneller oder langsamer fermentiert.

Allgemein anerkannt ist, dass Präbiotika durch die Erhöhung der Anzahl und Aktivität von Bifido- und Milchsäurebakterien die Darmgesundheit von Tier und Mensch als Gastorganismus verbessern. Dabei zählen zu den positiven Auswirkungen der Bifido- und Milchsäurebakterien (Lactobazillen) auf den Darm des Wirts vor allem die Verbesserung der Verdauung einschließlich einer verbesserten Nährstoff- und Mineralstoffabsorption sowie die Wirksamkeit und die intrinsische Stärkung des Immunsystems. Bifidobakterien stimulierende Produkte werden als bifidogene Faktoren bezeichnet.

Typische Nahrungsquellen für Präbiotika sind kohlenhydratspeichernde Pflanzen wie beispielsweise Sojabohnen und viele Getreidesorten, Topinambur- und Chicorée-Wurzeln sind z. B. Inulin-reiche Quellen.

Hinsichtlich der idealen täglichen Portion an Präbiotika gibt es unterschiedliche Empfehlungen. Typischerweise liegen diese im Bereich von 4-8g für die Unterstützung der allgemeinen Gesundheit des Verdauungssystems. Bis zu 15g oder mehr sind bei starken Verdauungsstörungen angeraten.

Aufgrund ihrer gesundheitlichen Vorteile werden präbiotische Oligosaccharide vermehrt zu Lebensmitteln beigefügt. Dies sind insbesondere Fructooligosaccharide (FOS), Xylooligosaccharides (XOS), Polydextrose und Galaktooligosacchariden (GOS). Mannooligosaccharide werden oft in Heimtierfutter für diese präbiotische Zwecke verwendet.

In der Patentschrift WO2010023422 (A1) - "Use of prebiotic Galacto-Oligosaccharides in the treatment of intestinal inflammation" von TZORTZIS ET AL. werden bereits in-vivo Daten im DSS-Mausmodell zu Präbiotika vorgestellt. Auch in der WO2006111624 (A1) - "Anti-inflammatory and/or analgesic composition for the intestine comprising branched maltodextrine" von WILS ET AL. werden Fructo-Oligosaccharide auf ihre anti-inflammatorische Wirkung im Mausmodell erfolgreich untersucht. Diese sind heute unter den Bezeichnungen ACTILIGHT, FIBERSOL, NUTRIOSE oder RAFTILOSE auf dem Rohstoffmarkt verfügbar.

Viele dieser Präbiotika kommen als natürliche Pflanzenkohlenhydrate vor, einige werden technisch modifiziert um die Eigenschaften für diätetische Zwecke anzupassen. Dazu werden z. B. Getreidestärken durch lebensmitteltechnologische Prozesse, wie Erhitzen, Säure- oder enzymatisch Behandlung in mehr oder weniger verdauungsresistente Homoglukane bzw. Dextrine umgewandelt, die sehr gut als präbiotische Ballaststoffe einsetzbar sind. Neuerdings haben sich auch gentechnischen Herstellungsverfahren von Inulinen etabliert.

Präklinische und klinische Studien zeigen positive Auswirkungen auf die Mineralien-Absorption, insbesondere die von Calcium, Hypertonie (Bluthochdruck), chronisch-entzündlichen Darmerkrankungen (Morbus Crohn und Colitis ulcerosa), Verringerung der Darmkrebsrisiko, allgemeine Verdauungsförderung und intestinale Regelung des Darm-pH sowie eine starke Wirksamkeit auf das Immunsystem und seine Stimulierung.

Diese vielseitigen gesundheitlichen Wirkungen werden durch die erhöhte Produktion von kurzkettigen Fettsäuren (SCFA; short chain fatty acids) aus den präbiotischen Oligosacchariden durch die stimulierten nützlichen Darmbakterien erklärt.

Da eine Aufnahme von großen Mengen von Präbiotika mit der Nahrung vorübergehend zu einem Anstieg der Gas-, Blähungen oder Stuhlgang führen kann, sollte die Einnahme von Präbiotika-haltigen Lebensmittel entsprechend langsam gesteigert werden, damit sich eine gesunde Bakterienflora schrittweise etablieren werden.

Die eingesetzten Präbiotika sind erfindungsgemäß ausgewählt aus der Gruppe der wasserlöslichen Kohlenhydrate. Bevorzugt können diese als Trocknungshilfsstoff eingesetzt und unterstützen somit die Trocknung und die Wirksamkeit. Die Verwendung von ACTILIGHT®, FIBERSOL®, NUTRIOSE® oder RAFTILOSE® ist besonders bevorzugt.

In der Medizin wird chronisch entzündlichen Darmerkrankungen (IBD) als eine Gruppe von entzündlichen Erkrankungen des Dickdarms und des Dünndarms definiert. Die wichtigsten Hauptformen von IBD sind Morbus Crohn und Colitis ulcerosa (UC).

Weit seltenere Arten, ebenfalls zu den chronisch entzündlichen Darmerkrankungen zu zählen, sind Kollagene Kolitis, lymphatischer Kolitis, ischämische Kolitis, Diversionskolitis, Behçet-Krankheit und nicht-klassifizierbare Kolitis.

Morbus Crohn und Colitis ulcerosa unterscheiden sich hauptsächlich aufgrund ihrer Lage und Beschaffenheit der entzündlichen Veränderungen. Morbus Crohn kann jeden Teil des Magen-Darm-Traktes befallen, vom Mund bis zum Anus, meist jedoch im terminalen Ileum beginnend. Colitis ulcerosa ist dagegen auf den Dickdarm und das Rektum beschränkt.

Histologisch ist Colitis ulcerosa auf die Schleimhaut (Darmepithel) beschränkt, während Morbus Crohn infolge transmuraler Läsionen die gesamte Darmwand betrifft.

Oft sind Morbus Crohn und Colitis ulcerosa mit nicht-Darm-Symptomen assoziiert, wie z. B. Lebererkrankungen, Arthritis, Hauterscheinungen und Augenproblemen. Auch lässt sich anfänglich nur schwer eine eindeutige Diagnose von Morbus Crohn oder Colitis ulcerosa stellen, so dass man von einer nichtklassifizierbaren Kolitis spricht.

Darüber hinaus treten diese Erkrankungen mit den folgenden sehr unterschiedlichen Symptomen auf: Bauchschmerzen, Erbrechen, Durchfall, rektale Blutungen, schwere innere Krämpfe und Muskelkrämpfe im Beckenbereich, Gewichtsverlust und die verschiedenen erwähnten assoziierten Beschwerden oder Krankheiten wie Arthritis, Pyoderma gangraenosum und die primär sklerosierende Cholangitis. Die endgültige Diagnose erfolgt in der Regel durch eine Koloskopie mit Biopsie der pathologisch veränderten Darmbereiche.

Die optimale Therapie von chronisch entzündlichen Darmerkrankungen hängt im Allgemeinen von der vorliegenden IBD-Erkrankungsart ab. Jedoch wird z. B. Mesalazin (5-ASA) oder das entsprechende Prodrug Sulfasalazin bei Colitis ulcerosa als auch bei Morbus Crohn verabreicht. Grundsätzlich kommen, je nach Symptomschweregrad der IBD Immunsuppressiva, wie Prednison, TNF-Hemmer sowie die Zytostatika Azathioprin, Methotrexat, oder 6-Mercaptopurin zum Einsatz. In schweren Fällen kann eine Operation erforderlich werden, wie z. B. eine partielle Darmresektion, Strikturoplastik oder eine temporäre oder permanente Kolostomie oder Ileostomie.

In der Regel wird die Behandlung durch Verabreichung von Medikamenten mit hohem entzündungshemmendem Effekt, wie Prednison gestartet. Sobald die Entzündungshemmung erfolgreich war, wird der Patient mit einem leichteren Medikament, wie Mesalazin behandelt, um eine Krankheitsremission zu erzielen. Wenn dies erfolglos ist, kann eine Kombination mit den oben genannten Immunsuppressoren mit Mesalazin, das auch eine entzündungshemmende Wirkung entfaltet, verabreicht werden.

Obwohl IBD die Lebensqualität aufgrund von Schmerzen, Erbrechen, Durchfall und andere sozial inakzeptablen Symptomen stark einschränkt, verläuft die Erkrankung alleine nur selten tödlich. Todesfälle aufgrund von Komplikationen wie toxisches Megakolon, Darmperforation und chirurgische Komplikationen sind ebenfalls selten. Zwar haben IBD-Patienten ein erhöhtes Risiko für Darmkrebs, aber aufgrund der Routine-Überwachung des Dickdarms wird eine solche Krebserkrankung sehr viel früher als bei der allgemeinen Bevölkerung durch eine Koloskopie entdeckt und behandelt.

Das Ziel der Behandlung nach Erreichen einer Remission ist es, leichtere Medikamente mit weniger potentiellen Nebenwirkungen zu verwenden. Zwischen diesen Monate oder Jahre dauernden Remissionsphasen kann es jederzeit zu Schüben, d.h. akuten Wiederauftreten der ursprünglichen Symptome kommen, die meist mehrere Wochen vorherrschen können.

Seit einiger Zeit wird über neue Therapiestrategien berichtet, die bei vielen Formen der chronisch entzündlichen Darmerkrankungen vielversprechend zu sein scheinen.

Erste Berichte legen nahe, dass eine "helminthische Therapie", bei der Darmparasiten, wie Trichuris suis oder der Hakenwurm Necator americanus verabreicht werden, sich sehr positiv auf die IBD-Symptome auswirkt, wobei spekuliert wird, dass neben der Stimulierung des Immunsystems ein länger anhaltender "Immunisierungs"-Effekt für die Heilwirkung verantwortlich ist.

Besonders durch den Einsatz von Präbiotika und Probiotika verspricht man sich effektive Behandlungen der IBD und in einigen Studien haben sie sich als ebenso wirksam wie verschreibungspflichtige Medikamente gezeigt.

Cannabispräparaten wird ein ebenso positiver Einfluss auf die IBD-Symptomatik zugeschrieben. Die Cannabispflanze enthält mehr als 50 sogenannte pflanzenspezifische Cannabinoide, von denen Δ9-Tetrahydrocannabinol (THC) und Cannabidiol (CBD) die bekanntesten und aktivsten zu sein scheinen. Cannabinoide haben eine starke entzündungshemmende Wirkung, vor allem durch den CB2-Rezeptor. Dies konnte auch in präklinischen Untersuchungen an Nagern festgestellt werden. Des Weiteren wurde beobachtet, dass die zellvermittelte Immunität in chronischen Cannabiskonsumenten beeinträchtigt ist. Das Studium der funktionalen Rolle des Endocannabinoid-Systems bei der Immunomodulation zeigt, dass es an fast allen immunologischen Aktivitäten beteiligt ist. Cannabinoide verschieben das Gleichgewicht von proinflammatorischen Zytokinen und anti-inflammatorische Zytokinen hin zu den T-Helferzellen vom Typ 2 Profil (Th2-Phänotyp) und unterdrücken zellvermittelte Immunität, während humorale Immunität verstärkt wird.

Das Reizdarmsyndrom (RDS, IBS) ist eine Erkrankung des gesamten Verdauungstraktes, die Bauchschmerzen und Blähungen sowie Verstopfung oder Durchfall verursacht, wobei eine Vielzahl von Substanzen und emotionale Faktoren Symptome von IBS auslösen können.

In der Regel diagnostiziert ein Arzt IBS aufgrund der Symptome, nachdem er andere Darmprobleme ausgeschlossen hat, zum Beispiel durch Blut-, Stuhluntersuchungen und einer Sigmoidoskopie zur Differentialdiagnose zu IBD (Morbus Crohn, Colitis ulcerosa), Krebs und anderen chronifizierten Darmentzündungen.

Symptome sind Bauchschmerzen, die im Zusammenhang mit dem Stuhlgang (Defäkation) stehen und sich meist danach bessern. Veränderung der Stuhlfrequenz (wie z.B. Verstopfung oder Durchfall) oder -konsistenz, Bauch-Expansion (Ausdehnung), Schleim im Stuhl, und das Gefühl der unvollständigen Entleerung nach dem Stuhlgang sind weitere Probleme.

Eine gesunde und regelmäßige Ernährung steht beim Reizdarmsyndrom im Vordergrund, zusätzlich können Medikamente spezifische Symptome lindern.

IBS betrifft etwa 10 bis 20% der allgemeinen Bevölkerung in den Industriestaaten (z.B. in Mexiko bis 50%), wobei Frauen mit IBS eher einen Arzt aufsuchen. In Deutschland alleine soll es 7 Millionen Betroffene geben. IBS ist somit die häufigste Erkrankung, die durch Gastroenterologen diagnostiziert wird. IBS gehört mit dem Reizmagen (funktionelle Dyspepsie) zu den funktionellen Magen-Darmerkrankungen.

Die Ursache des Reizdarmsyndroms ist unklar. Bei vielen Menschen mit IBS ist der Verdauungstrakt besonders empfindlich auf viele Reize. Hierbei nimmt man Störungen im abdominalen Neuroplexus oder seiner Kommunikation mit dem ZNS an. Des Weiteren spielen emotionale Faktoren (z.B. Stress, Depression und Angst) sowie Ernährung, Medikamente, Hormon-schwankungen oder nur geringfügige Irritationen eine große Rolle und können IBS-Symptome auslösen oder verschlimmern.

Da die meisten Menschen mit IBS körperlich gesund erscheinen, ging man in der Vergangenheit beim IBS eher von einer psychosomatischen Ursache aus, doch wurden neuerdings an der Technischen Universität München (TUM) "Mini-Entzündungen" entdeckt und damit zusammenhängende neuronale Kommunikationsstörungen im Nervenkomplex des Darms als Ursache diskutiert. Diese Entzündungsherde bringen IBS in näheren Zusammenhang mit IBD, was gegebenenfalls ebenso eine entzündungshemmende Therapie des Reizdarms analog zum IBD eröffnet.

(1. Bercik P, Verdu EF, Collins SM. Is irritable bowel syndrome a low-grade inflammatory bowel disease? Gastroenterol Clin North Am. (2005) Vol. 34, Issue: 2: 235-45 // 2. Mearin F, Perelló A, Balboa A; Irritable bowel syndrome and inflammatory bowel disease: Is there a connection? Gastroenterologia y hepatologia (2009)Volume: 32, Issue: 5, Pages: 364-372 // 3.Quigley EMM, Shanahan F; Irritable Bowel Syndrome and Inflammatory Bowel Disease: Is There an Overlap? Practical Gastroenterology, November 2010, 31-37).

Die Behandlung ist personenspezifisch und besteht zunächst in der Vermeidung oder Ausschaltung der Ursache, wenn beispielsweise bestimmte Lebensmittel oder Stressarten das Problem auslösen. Liegt zusätzlich eine Verstopfung vor, so helfen oft regelmäßige körperliche Aktivitäten, um eine normale Verdauung wiederherzustellen. Grundsätzlich lässt sich ein IBS relativ gut durch eine entsprechende Ernährungsumstellung oder Diät positiv beeinflussen.

Herrschen Verstopfungen vor, so hilft oft die Zufuhr von mehr Ballast- und Faserstoffen oder anderen verdauungsfördernden Mitteln, wie Kleie, Indischer Flohsamen (Psyllium, Metamucil), Methylcellulosen, Sorbite, Lactulose, Constulose, Polyethylenglycole, Glycerin und Bisacodyl (DULCOLAX) oder Lubiprostone (AMITIZA). Darüber hinaus ist für Flohsamen auch eine direkte Wirksamkeit bei IBD und IBS postuliert worden (Baljit Singh: Review - Psyllium as therapeutic and drug delivery agent; International Journal of Pharmaceutics 334 (2007) 1-14).

Relaxantien für die glatte Muskulatur des Darms, wie Dicyclomin (BENTYL) oder Butylscopolamin (BUSCOPAN) können die Bauchschmerzen und -krämpfe lindern. Herrschen dagegen Durchfälle vor, so sollten Antidiarrhoika, wie das Opiatanalogon Loperamide (IMODIUM) oder das pflanzliche UZARA - ein Extrakt aus Xysmalobium undulatum- eingesetzt werden. Bei mit Übelkeit verbundenen Durchfällen kann Alosetron (US-Handelsname: LOTRONEX), ein selektiver Hemmstoff der 5-HT3-Rezeptoren zum Einsatz kommen. Vergleichbare antiserotonerge Wirkungen werden neuerdings dem Ingwer zugeschrieben.

Zur Behandlung von IBS-Symptomen kommen auch Antidepressiva, sowie Techniken zur Verhaltensmodifikation (wie z.B. kognitive Verhaltenstherapie), Psychotherapie und Hypnose zum Einsatz und zeigen oft eine gute Wirksamkeit. Auch ein langfristiger Einsatz von Antidepressiva ist relativ sicher und Antidepressiva können nicht nur Schmerzen und andere Symptome mindern, sondern auch Schlafstörungen, Depressionen oder Angstzustände lindern.

Neben diesen Therapien mit chemischen Pharmazeutika kommen immer öfter auch phytotherapeutische Präparate zum Einsatz. So insbesondere aromatische Öle wie Pfefferminzöl, die bei IBS-Symptomen, wie Blähungen und Bauchkrämpfen sehr effektiv sind.

Des Weiteren sind Probiotika und Präbiotika zu einem wichtigen Therapiebestandteil von IBS wie auch bei IBD geworden (Damaskos & Kolios: Probiotics and prebiotics in inflammatory bowel disease: microflora 'on the scopé ; 2008 Br J Clin Pharmacol / 65:4 / 453-467). In den Bereich der Präbiotika fallen darüber hinaus auch viele Pflanzenextrakte enthaltend sogenannte "soluble fibers", wie z.B. ein ballaststoffreicher Kladodien-Extrakt aus Opuntia ficus indica L. Diese wasserlöslichen Kohlenhydrate entlasten den Gastrointestinaltrakt, z. B. in dem Lipide emulgiert und dadurch retardiert freigesetzt werden, aber auch in dem diese Kohlenhydrate selbst nur von einigen erwünschten Bakterienspezies der Darmflora verstoffwechselt werden können und somit zu einer Verschiebung der Anteilshäufigkeit einzelner Bakterienspezies beitragen.

Die Erfindung wird durch die nachfolgenden nicht einschränkenden Beispiele näher erläutert.

### Beispiele

### Methoden

### Bestimmung Rosmarinsäure

Die quantitative Bestimmung der Rosmarinsäure erfolgt mittels Gradienten-HPLC an einer RP18-Phase (bevorzugt LiChrospher 100, RP 18, 5 µm, 250 x 4 mm), mit Detektion bei 320nm. Das binäre Elutionsmittel besteht aus A (15 Vol.% Acetonitril + 0,425% Phosphorsäure +84,575% Wasser) und B (80 Vol.% Acetonitril + 0,425% Phosphorsäure +19,575% Wasser) und wird im Rampenprofil mit 1ml/min von 100% A innerhalb von 40 Minuten auf 30% A + 70% B gefahren. Das Injektionsvolumen beträgt 10µl.

Als Referenz werden 25mg Rosmarinsäure genau eingewogen und in 100ml Methanol gelöst; hiervon werden 10ml auf 100ml verdünnt.

Für die Testlösung werden 400mg Thymian-Extrakt in einem 100ml-Messkolben mit 25ml Wasser und 25ml Methanol im Ultraschallbad gelöst. Der Messkolben wird nach der thermischen Equilibrierung auf RT mit 50%igem Methanol bis zur Marke aufgefüllt und gut durchgemischt. 10ml der Lösung werden auf 100ml mit 50%igem Methanol verdünnt.

### Bestimmung Thymol Carvacrol

Die quantitative Bestimmung der ätherisch Öl-Substanzen Thymol und Carvacrol erfolgt mittels Gaschromatographie. Hierzu wird eine Fused-Silica-Kapilarsäule (z.B. Fa. Zberon ZB-FFAP, code 7JM-G009-17) mit 50m Länge und einem Innerdurchmesser von 0,32mm verwendet. Als Trägergas kommt Wasserstoff 5.0 mit einem Fluss von 2,5mL/min zum Einsatz. Ein FID-Detektor (250°C) ist zur Erfassung geeignet. Die GC-Arbeitsbedingungen sind: 140°C (1 min isotherm), Aufheizen von 140°C bis 190°C mit 5°C/min, anschließend 190°C (5 min isotherm), weiteres Aufheizen von 190°C bis 220°C mit 20°C/min, anschließend 220°C (9 min isotherm).

Als Referenzsubstanzen werden reines Thymol (Merck 108167) bzw. Carvacrol (Fluka 22051) - je 50,0 mg - zusammen mit einer Internen-Standardsubstanz (50,0 mg 4-Isopropylphenol) in einen 50 ml Messkolben gegeben und mit n-Hexan bis zur Marke aufgefüllt.

Eine separate Lösung von 250,0 mg 4-Isopropylphenol als Interne-Standardsubstanz wird mit n-Hexan gelöst und aufgefüllt in einem 250 mL Messkolben.

Thymianextrakte werden mit ca. 1g eingewogen und in 40mL Methanol 30% V/V gelöst. Nach Überführung in einen 250 mL Scheidetrichter werden 5 ml der Internen Standardlösung zugegeben. Die gesamte Lösung wird dreimal mit je 40 mL Diethylether ausgeschüttelt. Die vereinigten Diethylether-Phasen werden über Natriumsulfat getrocknet, klar filtriert und am Rotationsverdampfer bei 40°C im leichten Vakuum beigedampft. Der Rückstand wird in 5,0 mL Ethanol 96% aufgenommen. Hiervon wird 1µl injiziert (Temperatur des Injektors 230°C).

Das GC-Chromatogramm enthält für Quendelextrakte Carvacrol als dominanten Peak (Rt ca. 12,7 min), wohingegen das Thymol ca. ein Drittel geringere Peakfläche aufweist (Rt ca. 12,1 min). Der interne Standard 4-Isopropylphenol liegt mit Rt ca. 12,9 min bei dieser Peak-Gruppe.

### Beispiel 1: wässriger Thymianextrakt (Stand der Technik)

14,3kg gerebelte und getrocknete Droge Herba Thymi Ph.Eur. (Thymus vulgaris) werden in einem Holstein-Kappert-Perkolator für 1,5 h mit überspanntem Wasserdampf von 134°C bedämpft, bis in der Abluft kein Geruch nach ätherischen Komponenten mehr festgestellt wird (<0,5ml/kg Droge). Anschließend wird mit 286 Litern Osmosewasser bei 60°C im Perkolator extrahiert. Das Eluat wird über die Droge abgelassen und über einen Siebbeutel mit 250µm Porengröße von Drogenresten befreit. Durch einen Plattenverdampfer wird das Eluat zu einem Dickextrakt mit ca. 53% Trockensubstanzanteil eingeengt. Von dem erhaltenen Spissum werden 70% Trockenextraktäquivalent mit 30% Maltodextrin Ph.Eur. versetzt, für 15 Minuten bei 121°C autoklaviert und bei 50°C im Vakuumtrockenschrank getrocknet. Der erhaltene Extrakt ist charakterisiert durch ein DEVnativ von 4 : 1, einen Gehalt an ätherischem Öl von <0,1%, hiervon <0,01% Thymol/Carvacrol und einen Gehalt an Rosmarinsäure von 2,5%.

### Beispiel 2: wässriger Quendelextrakt

17,8kg gerebelte und getrocknete Droge Herba Serpylli Ph.Eur. (Thymus serpyllum) werden in einem Holstein-Kappert-Perkolator für 1,5 h mit überspanntem Wasserdampf von 134°C bedämpft, bis in der Abluft kein Geruch nach ätherischen Komponenten mehr festgestellt wird. Anschließend wird mit 356 Litern Osmosewasser bei 60°C im Perkolator extrahiert. Das Eluat wird über die Droge abgelassen und über einen Siebbeutel mit 250µm Porengröße von Drogenresten befreit. Durch einen Plattenverdampfer wird das Eluat zu einem Dickextrakt mit ca. 50% Trockensubstanzanteil eingeengt.

### Beispiel 3: wässriger Quendeltrockenextrakt 1

Der nach Beispiel 2 erhaltene Spissum wird bei 50°C im Vakuumtrockenschrank nativ getrocknet. Der erhaltene Extrakt ist charakterisiert durch ein DEVnativ von 6 : 1, einen Gehalt an ätherischem Öl von <0,1%, hiervon <0,01% Thymol/Carvacrol und einen Gehalt an Rosmarinsäure von 2,6%.

### Beispiel 4: wässriger Quendeltrockenextrakt 2

Von dem nach Beispiel 2 erhaltenen Spissum werden 70% Trockenextraktäquivalent mit 30% NUTRIOSE FB 06 versetzt, für 15 Minuten bei 121°C autoklaviert und bei 50°C im Vakuumtrockenschrank getrocknet. Der erhaltene Extrakt ist charakterisiert durch ein DEVnativ von 6 : 1, einen Gehalt an ätherischem Öl von <0,1%, hiervon <0,01% Thymol/Carvacrol und einen Gehalt an Rosmarinsäure von 1,8%.

### Beispiel 5: 20% ethanolischer Quendelextrakt

500g gerebelte und getrocknete Droge Herba Serpylli Ph.Eur. (Thymus serpyllum) werden zweimal mit jeweils 5 Litern Ethanol 20% V/V bei 50°C im Perkolator extrahiert. Das Eluat wird über die Droge abgelassen und über einen Faltenfilter (32-WE) von Drogenresten befreit. Im Rotationsverdampfer wird unter scharfem Vakuum von <50 mbar das Eluat zu einem Dickextrakt mit ca. 50% Trockensubstanzanteil eingeengt. Der so erhaltene Spissum wird mit 30% NUTRIOSE FB 06 versetzt, für 15 Minuten bei 121°C autoklaviert und bei 50°C im Vakuumtrockenschrank getrocknet. Der erhaltene Extrakt ist charakterisiert durch ein DEVnativ von 6 : 1, einen Gehalt an ätherischem Öl von <0,1%.

### Beispiel 6: 40% ethanolischer Quendelextrakt

500g gerebelte und getrocknete Droge Herba Serpylli Ph.Eur. (Thymus serpyllum) werden zweimal mit jeweils 5 Litern Ethanol 40% V/V bei 50°C im Perkolator extrahiert. Das Eluat wird über die Droge abgelassen und über einen Faltenfilter (32-WE) von Drogenresten befreit. Im Rotationsverdampfer wird unter scharfem Vakuum von <50 mbar das Eluat zu einem Dickextrakt mit ca. 50% Trockensubstanzanteil eingeengt. Der so erhaltene Spissum wird mit 30% NUTRIOSE FB 06 versetzt, für 15 Minuten bei 121°C autoklaviert und bei 50°C im Vakuumtrockenschrank getrocknet. Der erhaltene Extrakt ist charakterisiert durch ein DEVnativ von 6 : 1, einen Gehalt an ätherischem Öl von <0,1%.

### Beispiel 7: 70% ethanolischer Quendelextrakt

500g gerebelte und getrocknete Droge Herba Serpylli Ph.Eur. (Thymus serpyllum) werden zweimal mit jeweils 5 Litern Ethanol 70% V/V bei 50°C im Perkolator extrahiert. Das Eluat wird über die Droge abgelassen und über einen Faltenfilter (32-WE) von Drogenresten befreit. Im Rotationsverdampfer wird unter scharfem Vakuum von <50 mbar das Eluat zu einem Dickextrakt mit ca. 50% Trockensubstanzanteil eingeengt. Der so erhaltene Spissum wird mit 30% NUTRIOSE FB 06 versetzt, für 15 Minuten bei 121°C autoklaviert und bei 50°C im Vakuumtrockenschrank getrocknet. Der erhaltene Extrakt ist charakterisiert durch ein DEVnativ von 6 : 1, einen Gehalt an ätherischem Öl von <0,1%.

### Beispiel 8: Quendel-CO2-Extrakt

500g gerebelte Droge Herba Serpylli Ph.Eur. (Thymus serpyllum) werden mittels überspanntem Wasserdampf bedämpft und weitgehend vom ätherischen Öl befreit. Die Droge wird getrocknet und auf Pulverform zerkleinert in eine Druckgasanlage überführt und dort mit 95% überkritischem Kohlendioxid und 5% Ethanol (als Modifier) bei 200 bar und 60°C für 60 Minuten extrahiert. Nach der Entspannung in das Produktaustragsgefäß resultierte ein Extrakt mit durchschnittlichem DEVnativ von 18 : 1 und einem Flavonoidgehalt von rund 2,3%. Der Gehalt an ätherischem Öl betrug nur 0,45% bezogen auf den trockenen nativen Extrakt. Der so erhaltene Extrakt wurde mit 30% FIBERSOL zu einem homogenen Extrakt verarbeitet und im Starmix gemahlen.

### Beispiel 9: in-vitro Testmodell

Lipopolysaccharid (LPS) -induzierte Freisetzung von Tumornekrosefaktor alpha (TNFα) in peripheren humanen Monozyten.

Humane primäre Monozyten werden in einer standardisierten Prozedur aus dem Leukozytenfilm von gesunden menschlichen Blutspendern gewonnen. Die Zellen werden für die ELISA-Tests in 24er Platten ausgesät.

Monozyten werden in der 24er Zellkulturplatte mit 10ng/ml LPS bei 37°C und 5 % CO₂ für 24 h stimuliert. Die Extrakte werden 30 min vor der LPS-Gabe hinzugefügt, um zu prüfen, ob sie die LPS-induzierenden Effekte verhindern können. Nach 24 h werden die Zellüberstände entnommen, zentrifugiert und auf TNFα Konzentrationen im ELISA gemäß Herstelleranweisungen (Biotrend, Germany; Immunotools, Germany) untersucht.

**Tabelle 1:**

| | TNFα-release @ 50µg/ml |
|---|---|
| Thymus vulgaris wäßrig (Bsp. 1) | 85% |
| Thymus serpylli wäßrig (Bsp. 4) | 58% |
| Thymus serpylli 20% EtOH (Bsp. 5) | 90% |
| Thymus serpylli 40% EtOH (Bsp. 6) | 62% |
| Thymus serpylli 70% EtOH (Bsp. 7) | 53% |

Es zeigt sich sowohl, dass wässriger Extrakt aus Thymus serpyllum einem wässrigen Extrakt aus Thymus vulgaris deutlich überlegen ist, als auch, dass neben dem rein wässrigen Extrakt auch höher ethanolische Extrakte von 40% bis 70% eine vergleichbar gute Inhibierung zeigen.

Das TNF-alpha mit den beanspruchten Erkrankungen in Zusammenhang steht, verdeutlicht die Literatur (Reinecker et al.: Enhanced secretion of tumour necrosis factor-alpha, IL-6, and IL-1fi by isolated lamina propria mononuclear cells from patients with ulcerative colitis and Crohn's disease; Clin Exp Immunol 1993; 94:174-181).

### Beispiel 10: in-vivo DSS-Testmodell

Diese Studie wurde in Übereinstimmung mit der Richtlinie zum Schutz der für Versuche und andere wissenschaftliche Zwecke verwendeten Tiere der Europäischen Union (86/609/EWG) durchgeführt.

Das Testsystem mittels DSS (= dextran sodium sulfate) in Mäusen eine Kolitis zu induzieren ist weitverbreitet und wissenschaftlich anerkannt.

In der vorliegenden Testserie werden 7-9 Wochen alte weibliche Mäuse (Stamm C57/BL6J) mit einem durchschnittlichen Körpergewicht von 20g eingesetzt, welche in klimatisierten Tierquartieren mit 12 stündigem hell-dunkel-Zyklus in Makrolonkäfigen gehalten werden, gefüttert mit Standard Nagetierfutter und Wasser ad libitum während des gesamten Experimentes. Die Mäuse werden zufällig zu 7 Gruppen (n=8) zugewiesen. Mit Ausnehme der Gesund-Kontroll-Gruppe erhalten alle Tiere von Tag d(-4) bis d(0) eine Konzentration von 3% DSS im Trinkwasser zur Induzierung der Kolitis. Anschließend wird wieder auf normales Trinkwasser umgestellt und der kurative Ansatz der Testreihe kann beginnen. Zwei Gruppen (Gesund- und Krank-Kontroll-Gruppe) erhalten oral lediglich das Verabreichungsmedium, die Versuchsgruppen erhalten entweder 100 mg/kg KG oder 250 mg/kg KG einer Thymianextraktzubereitung nach Beispiel 1, oder 100 mg/kg KG oder 250 mg/kg KG einer Quendelextraktzubereitung nach Beispiel 4, oder 50 mg/kg KG der chemischen Referenzsubstanz Sulfasalazin (SAZ). Die Substanzen werden jeweils als Lösung / Suspension über eine Magensonde für die Dauer von 7 Tagen verabreicht. An d(7) werden die Tiere mittels einer Überdosis Halothan getötet und der Darm für die Beurteilung der Darmschädigung entnommen. Ein Surrogatparameter der eng mit den entzündlichen Magen-DarmErkrankungen verbunden wird, ist IL 17 (1. Takanori Kanai et al.: Homeostatic (IL-7) and effector (IL-17) cytokines as distinct but complementary target for an optimal therapeutic strategy in inflammatory bowel disease, Current Opinion in Gastroenterology 2009, 25:306-313 // 2. Atsuhiro Ogawa et al.: Neutralization of interleukin-17 aggravates dextran sulfate sodium-induced colitis in mice, Clinical Immunology 110 (2004) 55- 62), der mittels RT-PCR bestimmt und auf die β-actin-Expression als internem Standard bezogen wird. Gleichfalls wurde mittels RT-PCR das interzelluläre Adhäsionsmolekül ICAM-1 bestimmt (1. R.C. Burns et al; Antibody blockade of ICAM-1 and VCAM-1 ameliorates inflammation in the SAMP-1/Yit adoptive transfer model of Crohn's disease in mice; Gastroenterology Volume 121, Issue 6 , Pages 1428-1436, December 2001 // 2. E. Rijcken et al; ICAM-1 and VCAM-1 antisense oligonucleotides attenuate in vivo leucocyte adherence and inflammation in rat inflammatory bowel disease; Gut 2002;51:529-535).

**Tabelle 2:**

| | Gesunde Kontrollgruppe | Kolitische Kontrollgruppe | Thymi serpylli 100mg/kgKG | Thymi serpylli 250mg/kgKG | Thymi vulgaris 100mg/kg KG | Thymi vulgaris 250mg/kg KG | Sulfasalazin 50mg/kg KG |
|---|---|---|---|---|---|---|---|
| IL-17 | 0,34 ±0,04 | 0,69 ±0,04 | 0,56* ±0,04 | 0,50* ±0,03 | 0,61 ±0,02 | 0,75 ±0,03 | 0,59* ±0,04 |
| ICAM -1 | 0,27 ±0,05 | 0,54 ±0,04 | 0,41* ±0,05 | 0,41* ±0,03 | 0,57 ±0,04 | 0,60 ±0,05 | 0,37* ±0,04 |

| | | | | | | | |
|---|---|---|---|---|---|---|---|
| * P < 0.05 vs. CONTROL | | | | | | | |

Hier ist zu erkennen, dass sowohl das mit IBD verknüpfte Zytokin IL-17 als auch das Integrin ICAM-1 durch den erfindungsgemäßen Quendelextrakt zu signifikant erniedrigten Ausschüttungen kommt, hingegen kann ein Thymus vulgaris Extrakt diesen Effekt nicht bestätigen.

### Beispiel 11: in-vivo TNBS-Testmodell

Diese Studie wurde in Übereinstimmung mit der Richtlinie zum Schutz der für Versuche und andere wissenschaftliche Zwecke verwendeten Tiere der Europäischen Union (86/609/EWG) durchgeführt.

Weibliche Wistar-Ratten (200-210 g) werden in Makrolonkäfigen gehalten, in klimatisierten Tierquartieren mit 12 stündigem hell-dunkel-Zyklus, gefüttert mit Standard Nagetierfutter und Wasser ad libitum während des gesamten Experimentes. Die Ratten werden zufällig zu 6 Gruppen (n=10) zugewiesen. Zwei Gruppen (Gesund- und Krank-Kontrolle) erhalten oral 1ml des Verabreichungsmediums, die Versuchsgruppen erhalten entweder 100 mg/kg KG einer Quendelextraktzubereitung nach Beispiel 4, oder 70 mg/kg KG einer Quendelextraktzubereitung nach Beispiel 3, oder 30 mg/kg KG des Präbiotikums NUTRIOSE oder in der Referenzgruppe 100 mg/kg KG Sulfasalazin (SAZ). Alle Testsubstanzen werden als Lösung, respektive Suspension über eine Schlundsonde für die Dauer von 7 Tagen einmal täglich verabreicht. Die Verabreichung der Testsubstanzen beginnt am gleichen Tag wie die Kolitis-Induktion. Zu diesem Zweck fasten die Ratten über Nacht und die Krank-Kontrollgruppe, als auch die Behandlungsgruppen werden dann mit TNBS wie folgt kolitisiert: Während einer Halothan-Narkose wird eine Lösung (10mg TNBS gelöst in 0,25ml Ethanol 50%V/V) über eine Teflonkanüle durch den Anus 8cm tief in den Darm eingespritzt. Den Ratten der Gesund-Kontrollgruppe werden 0,25ml einer PBS-Lösung anstelle der TNBS-Lösung verabreicht. Das Körpergewicht, die Wasser- und Nahrungsaufnahme, als auch die Stuhlkonsistenz werden während der Versuchsdauer täglich aufgezeichnet. Alle Ratten werden mittels einer Überdosis Halothan eine Woche nach Beginn der Colitis-Induktion getötet und der Darm für die Beurteilung der Darmschädigung entnommen. Der Darmabschnitt wird von Fett und Gekröse gesäubert und auf ein Filterpapier übertragen. Jede Probe wird gewogen und seine Länge unter konstanter Belastung (2g) vermessen, sowie daraus das Gewichts-Längen-Verhältnis ermittelt. Der Darm wird - unabhängig der verwendeten Behandlung - makroskopisch auf sichtbare Schädigung auf einer Skala von 0 bis 10 von zwei Beobachtern anhand zuvor festgelegter Kriterien bewertet, welche Umfang und Schwere der Schäden berücksichtigen. Ein Querschnitt des benachbarten Gewebes nahe des distalen erkrankten Bereiches wird unverzüglich mit 4%igem Formaldehyd fixiert und für histologische Untersuchungen in Paraffin eingebettet. Anschließend wird dieses Präparat für biochemische Bestimmungen in verschiedene Segmente aufgeteilt. Ein Teil wird bei -80°C zur Bestimmung der Myeloperoxidase (MPO) eingefroren, ein anderer Teil zur Glutathion-Bestimmung (GSH) in 5% Trichloressigsäure eingetaucht, und ein weiterer Teil unverzüglich zur Messung der Zytokinlevel von IL-6 und der iNOS-Expression verarbeitet. Schließlich wird das verbliebene Segment für die RNA-Extraktion und anschließende Analyse der Expression unterschiedlicher Marker mittels qPCR verwendet.

**Tabelle 3:**

| | **Macroskopischer Score** | **Microskopischer Score** | **MPO [mU/g]** | **GSH [nmol/g]** | **IL-6 [ng/g]** |
|---|---|---|---|---|---|
| **gesunde Kontrolle** | 0±0 | 0±0 | 155,1±74,8 | 1872,8±149,3 | 180,5±13,1 |
| **kolitische Kontrolle** | 7,8±0,2 | 28,1±2,5 | 8969,6±1402,0 | 877,4±107,6 | 281,8±26,8 |
| **Thymi serpylli Extrakt nach Beispiel 3 (70 mg/kg)** | 7,3±0,3 | 12,1 *±2,2 | 4629,3 *±705,5 | 1066,1±152,2 | 253,2±10,7 |
| **Nutriose (30 mg/kg)** | 7,6±0,4 | 23,6±3,6 | 8468,4±1461,5 | 977,6±116,4 | 246,5±19,9 |
| **Thymi serpylli Extrakt nach Beispiel 4 (100 mg/kg) Extrakt + Nutriose** | 6,6 *±0,4 | 9,6 *±3,9 | 4099,7 *±995,9 | 1490,8 *±176,2 | 207,5 *±18,1 |
| **Sulfasalazin (100 mg/kg)** | 6,9 *±0,4 | 13, 5 *±3,2 | 5591,5±537,0 | 1297,4 * ±184,3 | 222,0±16,1 |

| | | | | | |
|---|---|---|---|---|---|
| *= p < 0,05 vs. kolitische Kontrolle | | | | | |

Aus dieser Tabelle lässt sich ablesen, dass Nutriose in keinem der Parameter einen signifikanten Einfluss nehmen konnte. Dem gegenüber konnte der native Extrakt durch die Zugabe von Nutriose nicht nur an Potential gesteigert werden (mikroskopische Begutachtung, MPO-Aktivität), sondern wurde seinerseits in den Parametern makroskopischer Begutachtung, als auch dem GSH-Gehalt und der IL-6-Ausschüttung erst durch die Kombination mit Nutriose signifikant wirksam. Selbst das chemische Referenzpräparat Sulfasalazin konnte nicht in allen Parametern signifikante Verbesserungen zeigen.

Die Myeloperoxidase wird speziell in Zusammenhang mit chronischenzündlichen Erkrankungen gebracht (Tomohisa Saiki: Myeloperoxidase concentration in the stool as a new parameter of IBD; Kurume medical journal, 45,69-73,1998). Glutathion wird ebenfalls in der Literatur in Zusammenhang mit IBD bewertet (Sido et al.:Impairment of intestinal glutathione synthesis in patients with inflammatory bowel disease; Gut 1998 42: 485-492). Auch Interleukin 6 wird bereits in der Literatur mit Morbus Crohn und Colitis ulcerosa in Verbindung gebracht (Reinecker et al.: Enhanced secretion of tumour necrosis factor-alpha, IL-6, and IL-1fi by isolated lamina propria mononuclear cells from patients with ulcerative colitis and Crohn's disease; Clin Exp Immunol 1993; 94:174-181).

### Beispiel 12 - Messung zur Beeinflussung des Reizdarmsyndroms = Irretable Bowel Syndroms (IBS) im Tiermodell

Diese Studie wurde in Übereinstimmung mit der Richtlinie zum Schutz der für Versuche und andere wissenschaftliche Zwecke verwendeten Tiere der Europäischen Union (86/609/EWG) durchgeführt. Männliche Sprague Dawley -Ratten (240-320 g; Lieferant Janvier,St Berthevin Cedex) werden in Makrolonkäfigen mit 3-4 Tieren / Käfig gehalten, in klimatisierter Umgebung mit 12 stündigem hell-dunkel-Zyklus, gefüttert mit Standard Nagetierfutter und Wasser ad libitum während des gesamten Experimentes. Die Ratten werden zufällig zu vier Gruppen (n=10) zugewiesen. Alle Testsubstanzen werden als Lösung, respektive Suspension über eine Schlundsonde einmal täglich verabreicht.

Zwei Gruppen (Gesund- und Krank-Kontrolle) erhalten oral 1ml des Verabreichungsmediums; die Versuchsgruppen erhalten entweder 100 mg/kg KG einer Quendel-Extraktzubereitung nach Beispiel 4 oder 70 mg/kg KG an Gabapentin als Positivkontrolle. Gabapentin ist in der Literatur hierfür bereits beschrieben an Mäusen (Stepanovic-Petrovic RM, et al. The antinociceptive effects of anticonvulsants in a mouse visceral pain model. Anesth Analg. 2008; 106:1897-903) sowie an Ratten (0' Mahony SM, Coelho AM, Fitzgerald P, Lee K, Winchester W, Dinan TG, Cryan JF. The effects of gabapentin in two animal models of co-morbid anxiety and visceral hypersensitivity. Eur J Pharmacol. 2011; 667:169-74).

Die Ratten wurden narkotisiert mit Isofluran und eine Kanüle wurde durch den Anus ca. 6 cm tief in den Dickdarm eingeführt. Anschließend wird 1 ml 4 mM Desoxycholsäure (DCA) gelöst in Krebs'scher-Lösung bei pH 7,4 eingeflößt, während die Kanüle langsam zurückgezogen wird. Die Tiere wurden in einer "Kopf-unten-Position" belassen, um ein Austreten von DCA zu verhindern. Den Ratten wurden DCA einmal täglich an 3 aufeinanderfolgenden Tagen verabreicht. Die erste Injektion wurde als Tag 1 gezählt. Mäuse in der Kontrollgruppe erhielten 1 ml 0,9% iger Kochsalzlösung anstelle von DCA.

Die Bestimmung der kolorektalen Distension (CRD) erfolgte nach der Methode von La et al. (La JH, Sung TS, Kim HJ, Kim TW, Kang TM, Yang IS. Peripheral corticotropin releasing hormone mediates post-inflammatory visceral hypersensitivity in rats. World J Gastroenterol. 2008; 14:731-6). Die Gabe der Testsubstanzen begann 24 Stunden nach der letzten DCA-Verabreichung und wurde über 1 Woche durchgeführt. Anschließend wurden die verschiedenen Gruppen zur viszeralen Überempfindlichkeit gegen kolorektale Distension untersucht. Zu diesem Zweck wurden die Ratten über Nacht ohne Nahrung belassen (mit freiem Zugang zu Wasser), um die Ballon Platzierung zu erleichtern. Am Tag des Experiments wurden die Ratten kurz mit Isofluran betäubt und ein 5-6 cm Ballon, der an einem Schlauch hing, wurde durch den After in das Rektum und Colon descendens, das distale Ende 1 cm proximal zu dem äußeren Schließmuskel eingesetzt. Der Katheter wird dann an der Basis des Schwanzes angeklebt, um eine Verschiebung zu verhindern. Nach dieser Prozedur werden die Ratten in einer transparenten Kabine (20 cm x 8 cm x 8 cm) platziert und durften sich erholen. Nachdem die Tiere ganz wach und akklimatisiert sind, wurde die CRD durch Aufblasen des Ballons mit Luft produziert. Jeder Versuch bestand aus fünf definierten Stößen (60 mmHg) über 20s Dauer, 3-min Interstimulusintervall. Im Allgemeinen wurden 4 Versuche ausgeführt, um eine stabile Reaktion (weniger als 20% Variabilität zwischen den letzten beiden Versuchen) zu erreichen.

In diesem Experiment wurde der abdominale Rückzugs-Reflex (CRD) wie folgt bewertet:
0 = kein Verhaltensreaktion zu Blähungen,
1 = kurze Kopfbewegungen durch Immobilität,
2 = Kontraktion der Bauchmuskeln ohne Anheben des Bauches,
3 = Aufhebung des Abdomen,
4 = Körper wölbt sich und die Aufhebung der Becken-Struktur.

Anhand dieses Scores ließen sich folgende Ergebnisse nach 1 Woche Behandlung erhalten:

| Tiergruppe (n=10) | Gesunde Gruppe (kein IBS) (A) | Kranke Gruppe (IBS Kontrolle) (B) | Extrakt nach Bsp. 4 (T. serpylli) (C) | Positivkontrolle (Gabapentin) (D) |
|---|---|---|---|---|
| Mittelwert (Standardabweichung) | 0,25 (± 0,125) | 2,875 (± 0,25) | 0,375 (± 0,06) | 0,06 (± 0,03) |

Die Auswertung ergab, das mit DCA behandelte Ratten (B=kranke Kotrollgruppe) einen statistisch signifikanten Unterschied zu den unbehandelten Tieren (A=gesunde Kontrollgruppe) lieferten. Damit war das Modell grundsätzlich geeignet. Die Positivkontrolle Gabapentin (D) konnte die Literaturangaben bestätigen und war signifikant in seiner Wirkung (p<0,05) gegenüber der IBS-Kontrolle (B). Die Prüfsubstanz (C), der erfindungsgemäße Extrakt nach Bsp. 4, konnte seine Eignung zur Behandlung von IBS ebenfalls bestätigen. Der Thymi serpylli-Extrakt war signifikant niedriger im Vergleich zur IBS-Kontrollgruppe (B) (p<0,05) und auf dem gleichen Niveau wie die Tiere aus der gesunden Gruppe (A).

### Beispiel 13: Instant-Extraktzubereitung

Um im Rahmen einer Erhaltungstherapie die Zeit bis zum nächsten Rezidiv möglichst weit auszudehnen, bedarf es einer Applikationsform, die zu einer hohen Therapietreue führt. Dies ist beispielsweise mit Hilfe einer schnell zubereiteten Instant-Getränkezubereitung gut zu gewährleisten. Hierfür wird 50% Trockenextraktäquivalent eines Spissum nach Beispiel 3 mit 43% Nutriose FB 06, 5% Saccharose und 2% Aroma vermengt und mittels überkritischer Kohlensäure in einem Sprühturm zu einem Instantpulver getrocknet. Diese Zubereitung kann in Sachets abgefüllt werden und ist in kaltem Wasser leicht löslich. Die Aromatisierung kann den Bedürfnissen des Marktes leicht angepasst werden.

### Beispiel 14: Extraktzubereitung als "Astronautenkost"

Leider sind Morbus-Crohn- als auch Colitis-ulcerosa-Patienten häufig in einem gesundheitlich schlechten Zustand und nur noch zur Aufnahme von wenig klassischer Nahrung in der Lage. Für diese Klientel wird eine Extraktzubereitung zur Verfügung gestellt, die man umgangssprachlich als Astronautenkost bezeichnet, hinter der sich in der Regel eine hochkalorische nutrazeutisch bilanzierte Diät in Form einer 200ml-Trinkzubereitung oder einer Tube eines Geles verbirgt. Zur Herstellung eines solchen Gels wird eine Extraktzubereitung nach Beispiel 4 mit den sonstigen Inhaltstoffen (Vitamine, Mineralstoffe, Eiweiß und freie Aminosäuren) gemeinsam gelöst, homogenisiert und zu jeweils 80ml in Einwegtuben abgefüllt.

### Eine Tube (1 Portion) enthält danach:

1000mg Quendelextrakt, 6g Eiweiß, 30g Kohlenhydrate, davon 15g Zucker (Saccharose), 9g Fett, davon 1g gesättigte Fettsäuren, 44g Wasser, Mineralstoffe (96mg Natrium, 91mg Chlorid, 236mg Kalium, 174mg Calcium, 174mg Phosphor (Ca/P-Quotient 1,0), 33mg Magnesium), Spurenelemente (3,8mg Eisen, 2,9mg Zink, 430µg Kupfer, 32µg Iod, 16µg Chrom, 200µg Fluor, 800µg Mangan, 24µg Molybdän, 14µg Selen), Vitamine (240µg Vit.A, 400µg Vit.B1, 400µg Vit.B2, 4,3mg Niacin, 400µg Vit.B6, 64µg Folsäure, 1300µg Pantothensäure, 700µg Vit.B12, 9,6µg Biotin, 24mg Vit.C, 1,8µg Vit.D, 13µg Vit.K, ), 88mg Cholin.

## Patentansprüche

1. Extrakt aus Thymus serpyllum L. enthaltend zusätzlich ein Präbiotikum zur Verwendung bei entzündlichen Erkrankungen des Intestinalbereichs, ausgewählt aus Reizdarmsyndrom (IBS), entzündlicher Darmerkrankung (IBD), Lymphozytäre Colitis, Colitis ulcerosa, Divertikulitis, Duodenitis und Morbus Crohn, wobei das Präbiotikum aus der Gruppe der wasserlöslichen Kohlenhydrate ausgewählt wird.

2. Extrakt zur Verwendung nach Anspruch 1, wobei der Gehalt an Rosmarinsäure mindestens 0,5 Gew.-%, bezogen auf den nativen, getrockneten Extrakt, beträgt.

3. Extrakt zur Verwendung nach Anspruch 1 oder 2, wobei der Gehalt an Thymol plus Carvacrol < 0,01 Gew.-%, bezogen auf den nativen, getrockneten Extrakt, beträgt.

4. Extrakt zur Verwendung nach einem der Ansprüche 1 bis 3, wobei das Präbiotikum aus der Gruppe der Fructo-Oligosaccharide ausgewählt wird.

5. Verfahren zur Herstellung eines Extraktes mit folgenden Schritten:
a. Bereitstellen einer Droge aus oberirdischen Pflanzenteilen von Thymus serpyllum) L.
b. Optionalem Trocknen der Droge
c. Optionales Zerkleinern der Droge
d. Optionalem Rebeln der Blätter und Reduzierung des Stengelanteiles auf kleiner 2 Gew-%
e. Optionales Bedämpfen der Droge und Reduzierung des ätherischen Öles auf weniger als 0,5 ml/kg bezogen auf die getrocknete Droge
f. Extraktion der Droge mit Extraktionsmittel
g. Zumindest teilweises Entfernen des Extraktionsmittels unter Gewinnung eines Dickextraktes
h. Optionalem Entfetten mit flüssigen Lösemitteln
i. Optionale thermische Entkeimung
j. Hinzufügen eines Trocknungshilfsstoffes, wobei der Trocknungshilfsstoff ausgewählt wird aus der Gruppe der präbiotischen wasserlöslichen Kohlehydrate, besonders bevorzugt aus der Gruppe der Fructo-Oligosaccharide
k. Trocknung unter Erhalt eines Extraktes,
wobei bevorzugt mindestens einer der Schritte e, h oder i durchgeführt wurde.

6. Verfahren nach Anspruch 5, **dadurch gekennzeichnet, dass** das Extraktionsmittel ausgewählt ist aus der Gruppe Wasser, Alkohol, Keton, Ester, Ether oder überkritischer Gase oder deren Mischungen.

7. Verfahren nach Anspruch 5 oder 6, **dadurch gekennzeichnet, dass** die Extraktion bei einer Temperatur von 20°C bis 100°C durchgeführt wird, bevorzugt bei 50° - 90°C, besonders bevorzugt bei 60 - 80°C.

8. Verfahren nach einem der Ansprüche 5 bis 7, **dadurch gekennzeichnet, dass** die eingesetzte Droge der Monographie "Serpylli herba" der europäischen Pharmakopoe entspricht.

9. Verfahren nach einem der Ansprüche 5 bis 8, **dadurch gekennzeichnet, dass** die Trocknung eine Sprühtrocknung oder Unterdrucktrocknung ist.

10. Extrakt oder Extraktzubereitung erhältlich nach dem Verfahren gemäß einem der Ansprüche 5 bis 9.

11. Extrakt oder Extraktzubereitung nach Anspruch 10, **dadurch gekennzeichnet, dass** der Extrakt einen Rosmarinsäuregehalt von mindestens 0,5 Gew-% , bezogen auf den getrockneten Extrakt, aufweist, bevorzugt mindestens 1,5 Gew-%.

12. Extrakt oder Extraktzubereitung nach einem der Ansprüche 10 bis 11, **dadurch gekennzeichnet, dass** der Extrakt einen Gehalt an ätherischem Öl von höchstens 0,5 Gew-%, bezogen auf den getrockneten Extrakt, aufweist, bevorzugt höchstens 0,1 Gew-%, besonders bevorzugt höchstens 0,01 Gew-%.

13. Extraktzubereitung nach Anspruch 10, **dadurch gekennzeichnet, dass** der Trocknungshilfsstoff ausgewählt ist aus der Gruppe ACTILIGHT®, FIBERSOL®, NUTRIOSE® oder RAFTILOSE®.

14. Pharmazeutische Zubereitung, Medizinprodukt oder Lebensmittel enthaltend einen Extrakt oder eine Extraktzubereitung nach einem der Ansprüche 10 bis 13.

## Claims

1. An extract from *Thymus serpyllum* L., additionally comprising a prebiotic, for use in inflammatory diseases of the intestinal tract selected from irritable bowel syndrome (IBS), inflammatory bowel disease (IBD), lymphocytic colitis, ulcerative colitis, diverticulitis, duodenitis, and Crohn's disease, wherein said prebiotic is selected from the group of water-soluble carbohydrates.

2. The extract for use according to claim 1, wherein its content of rosmarinic acid is at least 0.5% by weight, based on the native dried extract.

3. The extract for use according to claim 1 or 2, wherein its content of thymol plus carvacrol is < 0.01% by weight, based on the native dried extract.

4. The extract for use according to any of claims 1 to 3, wherein said prebiotic is selected from the group of fructo-oligosaccharides.

5. A process for producing an extract, comprising the following steps:
a. providing a vegetable drug from aerial plant parts of *Thymus serpyllum* L.;
b. optionally drying said vegetable drug;
c. optionally comminuting said vegetable drug;
d. optionally destemming the leaves and reducing the stem content to less than 2% by weight;
e. optionally steaming the vegetable drug and reducing the essential oil to less than 0.5 ml/kg, based on the dried vegetable drug;
f. extracting the vegetable drug with an extractant;
g. at least partially removing the extractant to obtain a thick extract;
h. optionally defatting with liquid solvents;
i. optionally performing a thermal disinfection;
j. adding a drying aid, wherein said drying aid is selected from the group of prebiotic water-soluble carbohydrates, more preferably from the group of fructo-oligosaccharides;
k. drying to obtain an extract;
wherein preferably at least one of steps e, h or i is performed.

6. The process according to claim 5, **characterized in that** said extractant is selected from the group of water, alcohol, ketone, ester, ether or supercritical gases, or mixtures thereof.

7. The process according to claim 5 or 6, **characterized in that** said extraction is performed at a temperature of from 20°C to 100°C, preferably at from 50 °C to 90 °C, more preferably from 60 to 80 °C.

8. The process according to any of claims 5 to 7, **characterized in that** the vegetable drug employed corresponds to the monograph "Serpylli herba" in the European Pharmacopoeia.

9. The process according to any of claims 5 to 8, **characterized in that** said drying is spray-drying or vacuum drying.

10. An extract or extract formulation obtainable by the process according to any of claims 5 to 9.

11. The extract or extract formulation according to claim 10, **characterized in that** said extract has a content of rosmarinic acid of at least 0.5% by weight, based on the dried extract, preferably at least 1.5% by weight.

12. The extract or extract formulation according to either of claims 10 to 11, **characterized in that** said extract has a content of essential oil of at most 0.5% by weight, based on the dried extract, preferably at most 0.1% by weight, more preferably at most 0.01% by weight.

13. The extract formulation according to claim 10, **characterized in that** said drying aid is selected from the group of ACTILIGHT^{®}, FIBERSOL^{®}, NUTRIOSE^{®} or RAFTILOSE^{®}.

14. A pharmaceutical formulation, medicinal product or food product containing an extract or extract formulation according to any of claims 10 to 13.

## Revendications

1. Extrait de Thymus serpyllum L contenant en plus un prébiotique destiné à être utilisé en cas de maladies inflammatoires de la région intestinale, sélectionnées parmi le syndrome du côlon irritable (IBS), la maladie intestinale inflammatoire (IBD), la colite lymphocytaire, la colite ulcéreuse, la diverticulite, la duodénite et la maladie de Crohn, le prébiotique étant sélectionné parmi le groupe des glucides solubles dans l'eau.

2. Extrait destiné à être utilisé selon la revendication 1, la teneur en acide rosmarinique étant au moins égale à 0,5 %-poids, rapporté à l'extrait natif séché.

3. Extrait destiné à être utilisé selon la revendication 1 ou 2, la teneur en thymol plus carvacrol étant < 0,01 %-poids, rapporté à l'extrait natif séché.

4. Extrait destiné à être utilisé selon l'une des revendications 1 à 3, le prébiotique étant sélectionné parmi le groupe des fructo-oligosaccharides.

5. Procédé de production d'un extrait avec les étapes suivantes :
a. préparation d'un remède à partir de parties de plantes aériennes de Thymus serpyllum L
b. séchage optionnel du remède
c. broyage optionnel du remède
d. égrenage optionnel des feuilles et réduction de la proportion de tiges à moins de 2 %-poids
e. vaporisation optionnelle du remède et réduction de l'huile essentielle à moins de 0,5 ml/kg rapporté au remède séché
f. extraction du remède avec un agent d'extraction
g. enlèvement au moins partiel de l'agent d'extraction accompagné d'une production d'un extrait épais
h. dégraissage optionnel avec des solvants liquides
i. désinfection thermique optionnelle
j. addition d'un adjuvant de séchage, l'adjuvant de séchage étant sélectionné parmi le groupe des glucides solubles dans l'eau prébiotiques, de façon particulièrement préférée parmi le groupe des fructo-oligosaccharides
k. séchage tout en préservant un extrait,
de préférence au moins une des étapes e, h ou i ayant été effectuée.

6. Procédé selon la revendication 5, **caractérisé en ce que** l'agent d'extraction est sélectionné parmi le groupe eau, alcool, cétone, ester, éther ou gaz supercritiques ou leurs mélanges.

7. Procédé selon la revendication 5 ou 6, **caractérisé en ce que** l'extraction est réalisée à une température de 20°C à 100°C, de préférence à 50 ° à 90 °C, de façon particulièrement préférée à 60 à 80 °C.

8. Procédé selon l'une des revendications 5 à 7, **caractérisé en ce que** le remède utilisé correspond à la monographie « Serpylli herba » de la pharmacopée européenne.

9. Procédé selon l'une des revendications 5 à 8, **caractérisé en ce que** le séchage est un séchage par pulvérisation ou un séchage par aspiration.

10. Extrait ou préparation d'extrait pouvant être obtenue(e) suivant le procédé selon l'une des revendications 5 à 9.

11. Extrait ou préparation d'extrait selon la revendication 10, caractérisé(e) en ce que l'extrait présente une teneur en acide rosmarinique d'au moins 0,5 %-poids, rapporté à l'extrait sec, de préférence au moins 1,5 %-poids.

12. Extrait ou préparation d'extrait selon l'une des revendications 10 à 11, caractérisé(e) en ce que l'extrait présente une teneur en huile essentielle de 0,5 %-poids maximum, rapporté à l'extrait sec, de préférence de 0,1 %-poids maximum, de façon particulièrement préférée de 0,01 %-poids.

13. Préparation d'extrait selon la revendication 10, **caractérisée en ce que** l'adjuvant de séchage est sélectionné parmi le groupe ACTILIGHT®, FIBERSOL®, NUTRIOSOSE® ou RAFTILOSE®.

14. Préparation pharmaceutique, produit médical ou aliment contenant un extrait ou une préparation d'extrait selon l'une des revendications 10 à 13.
